# EUROPEAN PATENT APPLICATION

(11) **EP 1 570 857 A1**
(43) Date of publication of application: **07.09.2005**
(21) Application number: 04075631.4
(22) Date of filing: 27.02.2004
(51) Int. Cl.: A61K 39/395, A61P 35/00, A61P 37/00, C07K 16/28

(54) **Method for inhibiting the transendothelal migration of cells such as leukocytes or tumor cells by a CD81 binding agent and uses thereof**

(71) Applicant: Nederlandse Organisatie voor Toegepast-Natuurwetenschappelijk Onderzoek TNO, 2628 VK Delft (NL)
(72) Inventor: Dijkstra, Sipke, 3981 ZP Bunnik (NL); van Noort, Johannes, Maria, 2332 AT Leiden (NL)
(74) Representative: Prins, Adrianus Willem

(57) **Abstract**

The invention provides a method for inhibiting the transmigration of cells, such as leukocytes or tumor cells, across endothelial cells, by contacting said cells with a CD81 binding agent. Furthermore, the invention provides a method for retarding or inhibiting tissue damage in a subject suffering from an inflammatory disorder, or tumor metastasis, comprising administering to said subject a pharmaceutical composition containing a CD81 binding agent capable of inhibiting transmigration of cells, such as leukocytes and tumor cells.

## Description

### Field of the invention

The present invention is in the fields of therapy and pharmaceutical compositions. The invention relates to methods and means, in particular pharmaceutical products, for inhibiting the transmigration of cells from the bloodstream into a target tissue and for treating a pathological condition associated with such cell transmigration. By providing substances inhibiting or reducing transmigration of cells, such as leukocytes in particular, the invention provides new therapeutic approaches to various inflammatory disorders and/or tumor metastases of various organs and/or tissues in the mammalian body.

### Background of the invention

Leukocyte transmigration is the process whereby leukocytes migrate in and out of the vasculature to sites of inflammation, which is part of normal immune surveillance and host defence against infection. The cellular and molecular mechanisms underlying leukocyte transmigration have been studied extensively (for reviews see Springer 1994, Weber 2003, Luscinskas 2003).

The current model of leukocyte transmigration is that after initial contact or tethering, the leukocyte rolls along the endothelium. During rolling, rapid and transient adhesion contacts between the leukocyte and the endothelium are associated with active signalling which will lead to arrest and firm adhesion of the rolling leukocyte on the vessel wall. Upon firm adhesion, the leukocytes crawl across the vessel wall and subsequently squeeze in between tightly apposed endothelial cells to enter the underlying tissue (diapedesis), where they release chemical mediators to combat infection.

Adhesion molecules are critically involved in all steps of the transmigration process; these can be grouped into selectins, integrins and members of the immunoglobulin superfamily. Whereas the first stages of leukocyte transmigration (rolling and adhesion) have been studied extensively, less is known about the regulation of the later steps including diapedesis and the role of endothelial junctions therein.

Leukocyte transmigration not only occurs during normal inflammatory responses, but is also associated with several inflammatory disorders where leukocyte transmigration and subsequent production of toxic mediators may cause the destruction of otherwise normal tissue. For example, in patients with multiple sclerosis (MS) large numbers of leukocytes are found in the CNS parenchyma and their presence is associated with tissue damage. There are numerous other pathological conditions where tissue damage is associated with increased transmigration of leukocytes into a target organ or tissue. These include rheumatoid arthritis, Crohn's disease, stroke, traumatic brain or spinal cord injury, Alzheimer's disease, AIDS dementia, atherosclerosis, diabetes, myocard infarction, and tissue or organ transplantation (host-versus-graft and graft-versus-host disease). In addition, cellular transmigration is involved in tumor metastasis when tumor cells use the bloodstream to form metastases elsewhere in the body.

Such transmigration-related disorders may be treated by interfering with one or more steps in the transmigration process (i.e. tethering, rolling, firm adhesion, diapedesis), which will block the entry of leukocytes and thus prevent the release of toxic mediators in the tissue. In attempting to block transmigration, prior art has focussed on inhibiting the binding of leukocytes to the endothelial surface by direct targeting of adhesion molecules on the endothelium and their receptors on the leukocyte. This has led to an emerging class of new compounds known as selective adhesion molecule inhibitors (for review see Ulbrich 2003). The best example is natalizumab, an antibody that antagonises VLA-4, a leukocyte receptor for the adhesion molecule VCAM-1 present on the endothelial surface. VLA-4 antagonists can block leukocyte transmigration in vitro and in vivo in animal models, resulting in a reduction of disease severity in the latter (e.g. Yednock 1992; Meerschaert 1995). Natalizumab (Antegren®) is now being evaluated in clinical trials for multiple sclerosis (Miller 2003) and Crohn's disease (Ghosh et al. 2003).

Unfortunately, there is paucity in the number of lead targets being evaluated for the treatment of transmigration-related disorders. In addition, current leads are usually targeting a single adhesion molecule, whereas several different adhesion molecules are involved in leukocyte transmigration. The prior art does not provide targets that simultaneously regulate the actions of several different adhesion molecules. There is a need of other targets and especially targets which involve the actions of a plurality of adhesion molecules. The present invention addresses the above and other needs.

The biological molecule CD81 (also known as TAPA or TAPA-1) is a member of the tetraspanin family of small membrane proteins, composed of four conserved transmembrane domains and two extracellular loops. Within the plasma membrane of cells, tetraspanins are found in large complexes together with integrins, other tetraspanins and several other proteins. Tetraspanins are functionally implicated in cellular proliferation, motility, adhesion, and activation in a range of tissues. For reviews on tetraspanins see Hemler 2003, Boucheix 2001.

CD81 is broadly expressed in mammals, with relatively high levels in leukocytes and in glial cells of the central nervous system (CNS). The primary CD81-associated membrane proteins include the integrin and adhesion molecule VLA-4 (Mannion 1996), and the immunoglobulin superfamily proteins EWI-2 and EWI-F (Hemler 2003). Furthermore, CD81 associates with signalling molecules such as phosphatidylinositol 4-kinase and protein kinase C (Hemler 2001).

CD81 has multiple functions in various tissues. In the immune system CD81 is involved in the development of Th2 immune responses as well as in antigen presentation. In the brain, CD81 is involved in the control of glial cell numbers (Geisert 2002; Kelic 2001) and scar formation (Irwin 1993). It has been shown that a CD81 antibody inhibits the proliferation of rat astrocytes (Geisert 1996). Increased numbers of astrocytes are present in the brains of CD81 knockout mice where the astrocytes apparently have undergone increased proliferation (Geisert 2002). Furthermore, CD81 has been identified as a co-receptor for hepatitis C virus (Pileri 1998). Finally, there is evidence for involvement of CD81 in cellular motility (e.g. Yanez-Mo 1998; Penas 2000).

A large number of patent documents mentions CD81 and suggests a biomedical utility thereof. Some relate to the development of specific classes of CD81 binding agents. Thus, WO 02/02631 is related to structure-based drug design based on the crystal structure of CD81 in order to obtain a drug for treating hepatitis C infection. WO 03/040333 is related to the design of CD81 binding agents based on NrS1, a putative receptor protein for CD81 identified in neurons, useful in a method for inhibiting proliferation of astrocytes. Inhibition of astrocyte proliferation by CD81 itself is taught by WO 02/058709. Furthermore, WO 03/040333 teaches the use of CD81 to enhance the survival of neurons in neurode generative diseases including multiple sclerosis.WO 03/053342 teaches modulation of CD81 expression by antisense technology for the treatment of diseases associated with expression of CD81.

Other patent documents mention CD81 in a long list of markers for a particular cell type, such as B cells, neural stem cells and endothelial cells. For example, WO 00/67796 claim the utility of antagonists binding to B cell surface markers for treatment of autoimmune diseases. The list of examples of B cell markers includes CD81, but all experiments relate to the anti-CD20 antibody rituximab and no experimental detail is provided on the production and use of anti-CD81 antibodies. WO 02/086082 provides a list of neural stem cell markers including CD81 and proposes to use such markers in a method for enriching for neural stem cells. WO 03/084469 provides a list of endothelial cell markers including CD81 and proposes to use such markers for targeting a therapeutic complex to a selected tissue.

Some patent documents, e.g. WO 99/18198 and US 2003/0157132, relate to the use of CD81 in the diagnosis and treatment of hepatitis C virus infection.

WO 98/25647 (Beth Israel Deaconess Medical Center) claims the use of CD81-mediated signal transduction to interfere with mast cell activation and to treat allergic conditions including asthma, hay fever or atopic eczema.

None of these patent documents concern transmigration of leukocytes, or more specifically transmigration of T cells and monocytes.

It has been demonstrated that anti-CD81 antibodies can affect cellular motility induced by the in vitro scratching of confluent monolayers of epithelial or mesenchymal cells (Mazzocca 2002, Penas 2000, Yanez-mo 1998, Domanico 1997). There is also a report of CD81 antibodies affecting the in vitro motility of leukocytes, in this case of a mouse T cell line (Clark 2001). Nothing is known about CD81-mediated motility in other leukocytes, for example monocytes, B cells, natural killer cells, or polymorphonuclear cells. Available evidence suggests that CD81 influences cellular motility through an integrin-related mechanism (Berditchevski 2001). It appears that CD81 is critical for activation of VLA-4 during leukocyte adhesion to surfaces coated with a VLA4 ligand (Feigelson 2003).

The scientific literature described above on the involvement of CD81 in cellular motility over non-cellular substrates in vitro does not allow to draw conclusions about its role, if any, in leukocyte transmigration in vivo. Transmigration of leukocytes requires active signalling from the leukocyte to its substrate, the endothelium, and vice versa. For example, the transition from leukocyte rolling to arrest and firm adhesion is enhanced by chemokine secretion from the endothelium (e.g. Alon 2003). Furthermore, during diapedesis (i.e. the actual passing of the leukocyte between apposing endothelial cells) the endothelial cells have to open and close the tight endothelial lateral junctions. In addition, it has been suggested that leukocytes could even migrate through the cell body of an endothelial cell. Thus, in contrast to the case of cellular motility in vitro, the substrate (i.e. the endothelial cells) plays a very active role in leukocyte transmigration.

### Summary of the invention

This invention is based on the novel finding that CD81 is important for the transmigration of cells, such as leukocytes and tumor cells, and that such transmigration can be inhibited efficiently by targeting CD81.

The present invention proposes CD81 as a novel target to suppress leukocyte transmigration in a treatment for inflammatory disorders and other diseases associated with the transmigration of cells.

Disclosed is the surprising finding that an anti-CD81 antibody is capable of inhibiting the transmigration of leukocytes across a monolayer of endothelial cells *in vitro.* It is contemplated that the administration of anti-CD81 antibodies or other CD81-binding agents in a pharmaceutical composition *in vivo* can be used to treat subjects suffering from a range of inflammatory disorders. Furthermore, the method of the present invention may be used to prevent the transmigration of tumor cells in subjects suffering from malignant disease.

This invention provides for the use of a CD81 binding agent for manufacturing a medicament for inhibiting or reducing transmigration of cells, such as leukocytes and tumor cells.

Said CD81 binding agent is preferably selected from the group consisting of CD81-binding polyclonal, monoclonal, chimeric, humanized, and fully human antibodies; CD81-binding Fab, F(ab')₂ or other antibody fragments, CD81-binding single chain Fv's, CD81-binding CDRs or other CD81-binding peptides, aptamers and small molecules.

Said leukocytes preferably are T lymphocytes or monocytes.

Said medicament preferably is for use in the treatment or prevention of an inflammatory disorder associated with leukocyte transmigration, or tissue damage caused by such disorder. Said disorder preferably is one of multiple sclerosis, stroke, spinal cord injury, traumatic brain injury, meningitis, Alzheimer's disease, Parkinson's disease, AIDS dementia, atherosclerosis, diabetes, inflammatory bowel disease (including Crohn's disease and ulcerative colitis), ischemia-reperfusion injury (including ischemic stroke and myocard infarction), rheumatoid arthritis, osteoarthritis, psoriasis, complications of tissue or organ transplantation including graft-versus-host and host-versus-graft disease.

According to another preferred embodiment, said medicament is for use in the treatment or prevention of tumor metastasis associated with transmigration of tumor cells.

This invention also provides a method for inhibiting or reducing transmigration of cells, such as leukocytes or tumor cells, comprising contacting said cells with a CD81 binding agent.

Preferably, an effective amount of said CD81 binding agent is administered to a mammal suffering from, or being at risk of developing, an inflammatory disorder associated with leukocyte transmigration, or tissue damage caused by such disorder, or an effective amount of said CD81 binding agent is administered to a mammal suffering from, or being at risk of developing, tumor metastasis associated with transmigration of tumor cells.

### Brief description of the drawings

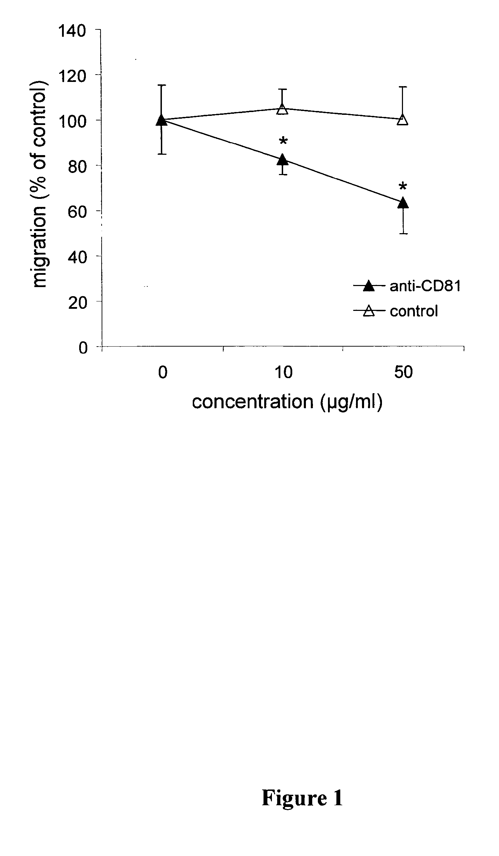

Figure 1 is a graph showing that leukocyte transmigration is inhibited by anti-CD81 antibody in vitro. NR8383 monocytes were allowed to transmigrate for 4 hours across confluent monolayers of GP8 endothelial cells, in the presence or absence of anti CD81 antibody or an irrelevant isotype matched control antibody at 10 or 50 µg/ml. Results are expressed as a percentage of the transmigration observed in the absence of antibody; data points represent mean ± SD (n=4). Asterisks indicate statistically significant differences (p<0.05). The graph clearly demonstrates a dose-dependent inhibition of monocyte transmigration by anti-CD81 antibody, with maximal inhibition at a concentration of 50 µg/ml. Furthermore, monocyte transmigration is not affected by the presence of control antibody.

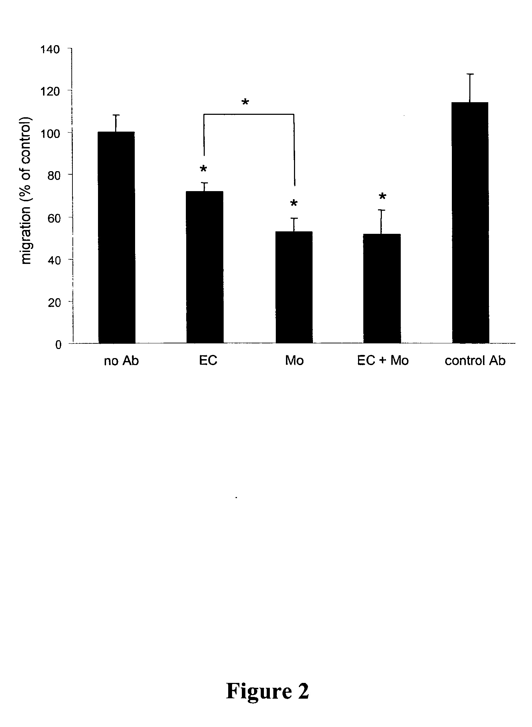

Figure 2 depicts a graph illustrating that the inhibitory effect of anti-CD81 antibody on leukocyte transmigration is predominantly, but not exclusively, mediated through CD81 present on the leukocytes as opposed to the endothelium. NR8383 monocytes and confluent monolayers of GP8 endothelial cells were separately preincubated with 50 µg/ml of anti-CD81 or control antibody for 30 min. Preincubation with anti-CD81 antibody was performed with either endothelial cells only (EC), with monocytes only (Mo), or with both monocytes and endothelial cells (EC+Mo). After preincubation, the antibodies were washed away and the monocytes were allowed to transmigrate across the endothelial cells for 4 hours. Results are expressed as a percentage of the transmigration observed in the absence of antibody; bars represent mean ± SD (n=4). Asterisks indicate statistically significant differences (p<0.05). The graph shows a significant inhibition of leukocyte transmigration when both endothelial cells and monocytes were treated with anti-CD81 antibody. Preincubation of monocytes alone resulted in a similar level of inhibition, whereas preincubation of endothelial cells alone was less inhibitory, although still significantly different from control treated cells.

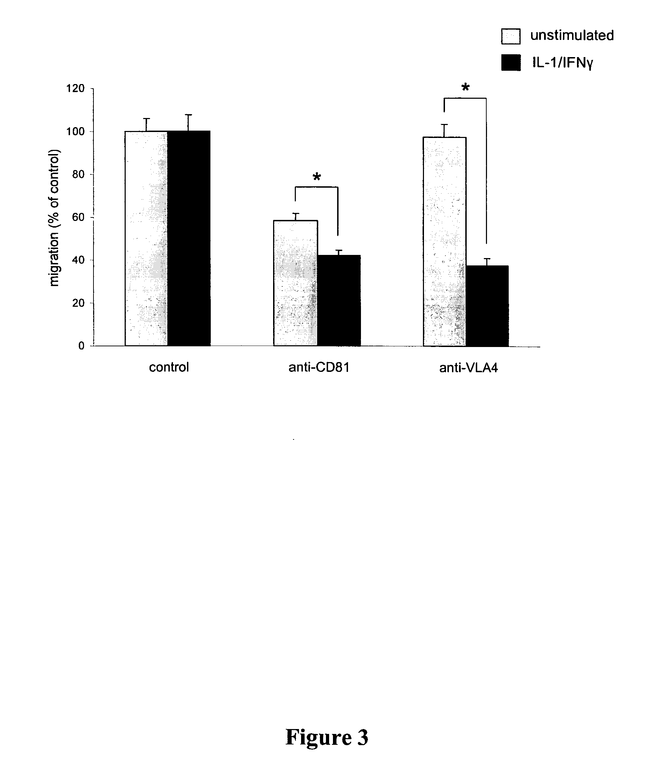

Figure 3 depicts the effect of pre-activation of the endothelial cells on the inhibitory effect of anti-CD81 antibody on leukocyte transmigration. NR8383 monocytes were allowed to transmigrate across monolayers of GP8 endothelial cells, which had been cultured for 48 hours with or without a combination of the proinflammatory cytokines IL1-β (100 ng/ml) and IFN_{Y} (200 U/ml). Transmigration was performed in the presence or absence of anti-CD81 antibody (50 µg/ml) or anti-VLA-4 antibody (10 µg/ml). The anti-VLA-4 antibody was included for comparison, since transmigration inhibition by this antibody is particularly sensitive to activation of the endothelial cells (Floris 2002). Results are given as a percentage of the level of transmigration observed in the absence of antibody. For non-activated endothelial cells (gray bars), 100% corresponds to 20 ± 1% migrated cells of the total number of added monocytes; for stimulated endothelial cells (black bars) the 100% value corresponds to 23 ± 2% migrated cells. The graph shows that activation of the endothelial cells further increased the inhibitory effect of anti-CD81 antibody. In addition, the level of inhibition observed on activated endothelial cells in the presence of anti-CD81 antibody is similar to that observed in the presence of VLA4 antibody.

### Detailed description of the invention

The present invention provides a method for inhibiting the transmigration of cells, such as leukocytes or tumor cells, comprising contacting said cells with a CD81 binding agent.

Additionally, the invention provides a method for treating a subject suffering from an inflammatory disorder that is associated with leukocyte transmigration.

The term 'leukocytes' refers to white blood cells, more in particular monocytes, T lymphocytes, B lymphocytes, natural killer (NK) cells, and polymorphonuclear cells. Most preferably, the leukocytes are T lymphocytes or monocytes.

As used herein, 'leukocyte transmigration' is defined as the process whereby leukocytes leave the bloodstream and enter into a target tissue, resulting in an increased number of leukocytes present within said target tissue. As used herein, 'inhibition of transmigration' refers to a partial or complete reduction in the number of leukocytes that is present in a target tissue after having crossed by any mechanism a biological barrier containing endothelial cells. In the context of the invention, the biological barrier may comprise either an in vitro system containing endothelial cells, or the wall of a blood vessel in a mammal in vivo. 'Mammal' as used herein includes man.

Furthermore, the method of the present invention may be used to prevent the transmigration of tumor cells in the process of metastasis.

It is contemplated that treatment with a pharmaceutical composition containing a CD81 binding agent will at least partially prevent leukocyte transmigration and thus reduce tissue damage in a subject suffering from an inflammatory disorder. Inflammatory disorders that may be treated with said pharmaceutical composition include, without limitation: MS (multiple sclerosis), stroke, spinal cord injury, traumatic brain injury, meningitis, Alzheimer's disease, Parkinson's disease, AIDS dementia, atherosclerosis, diabetes, inflammatory bowel disease (including Crohn's disease and ulcerative colitis), ischemia-reperfusion injury (including ischemic stroke and myocard infarction), rheumatoid arthritis, osteoarthritis, psoriasis, complications of tissue or organ transplantation including graft-versus-host and host-versus-graft disease. More preferably, the inflammatory disorder is MS or inflammatory bowel disease.

Additionally, the method of the present invention may be used to inhibit the transmigration of tumor cells in order to prevent tumor metastasis in a subject suffering from malignant disease.

Treatment according to this invention comprises both therapeutic and prophylactic treatment.

The invention thus provides a pharmaceutical composition for reducing or preventing leukocyte transmigration in a mammal, preferably a human, suffering from an inflammatory disorder. Said pharmaceutical composition comprises a CD81 binding agent together with a pharmaceutically acceptable carrier.

As used herein, a 'CD81 binding agent' is defined as a substance that binds to CD81 in such a way that this binding results in a modulation of the biological activity of the CD81-expressing cell. Preferably, the CD81-expressing cell is a leukocyte and the biological activity is transmigration as described above. The CD81 binding agent may be an antibody, specifically a monoclonal antibody, or a variant or part thereof capable of specific binding to CD81, or a peptide, small molecule or aptamer and the like, having the property of specific binding to CD81. Specific embodiments of CD81 binding agents will be described below.

### Antibodies and variants or fragments thereof

In a preferred embodiment the CD81 binding agent is an antibody to CD81 or a variant or antigen binding fragment of such antibody. Such antibodies include, but are not limited to, polyclonal, monoclonal, chimeric, humanized, and fully human antibodies; antibody fragments include , Fab, F(ab')₂ or single chain Fv fragments (scFv's). Antibodies to CD81 can be generated using methods that are well known in the art, e.g. for immunisation of mice. Monoclonal antibodies to CD81 can be prepared using any technique which provides for production of antibody molecules by continuous cell lines in culture. These include, but are not limited to, the hybridoma, the human B-cell hybridoma, and the EBV-hybridoma techniques.

In addition, techniques developed for the production of chimeric antibodies can be used [See, e.g., Pound (1998) Immunochemical Protocols, Methods Mol Biol Vol. 80].

Furthermore, humanised antibodies can be created e.g. by grafting the DNA encoding the antigen-binding loops (known as complementarity-determining regions or CDRs), from the DNA encoding a mouse monoclonal antibody into the DNA encoding a human IgG.

As an alternative to humanisation, fully human antibodies can be generated. These have high affinity for their respective antigens and can e.g. be obtained from very large, single-chain variable fragments (scFvs) or Fab phage display libraries. Alternatively, fully human antibodies can be obtained from transgenic mice that contain some, or preferably many, human immunoglobulin genes and genetically disrupted endogenous immunoglobulin loci. Immunization of such mice elicits the production of human antibodies recoverable using standard hybridoma technology as mentioned above.

Several techniques are known in the art for the production of antibody fragments. Fab or F(ab')₂ fragments, which contain the antigen binding sites, can e.g.be generated by proteolytic digestion of intact antibodies or directly produced by recombinant host cells. Alternatively, techniques described for the production of single chain antibodies (ScFv's) can be employed.

Various immunoassays can be used to identify antibodies having the desired specificity, i.e. CD81. Numerous protocols for binding, competitive binding or immunoradiometric assays using either polyclonal or monoclonal antibodies with established specificities are well known in the art.

### Peptides

In another embodiment the CD81 binding agent is a peptide. Techniques for peptide synthesis are well known in the art; additionally peptides with the desired amino acid sequence can be obtained commercially. The peptides as contemplated in the present invention may consist of between 6 and 50, preferably between 8 and 40 and more preferably between 10 and 30 amino acids.

In one embodiment the peptide may represent the antigen-binding site of a therapeutically effective anti-CD81 antibody. In general, an antibody comprises two types of polypeptides, the large H chains and the smaller L chains. Each polypeptide comprises a C-terminal 'constant' region and an N-terminal 'variable' region which forms the antigen binding site. The variable region is further divided into complementarity-determining regions 'CDRs' which are deeply involved in the formation of the antigen binding site, and 'frameworks' which are present in-between. Peptides to be used as CD81 binding agents will comprise amino acid sequence stretches of the CDRs of a therapeutically effective anti-CD81 antibody.

In another embodiment the amino acid sequence of the antibody is based on the E2 envelope protein of the hepatitis C virus. It is known that recombinant E2 protein binds to CD81 and that this binding results in modulation of the biological activity of the CD81 expressing cell, as shown for natural killer cells (Crotta 2002). Hence it is contemplated that peptides based on the E2 region that interacts with CD81 can be used as CD81 binding agents as envisaged in the invention.

In yet another embodiment the peptide may be selected from a phage displayed peptide library, for example as described by Cao et al (2003).

Various immunoassays can be used to identify peptides having the desired specificity, i.e. CD81. Numerous protocols for binding, competitive binding or immunoradiometric assays using either polyclonal or monoclonal antibodies with established specificities are well known in the art.

### Aptamers

Aptamers are synthetic DNA or RNA oligonucleotides which can interact with target molecules with very high specificity and affinity as a result of their complex three-dimensional structure (for review, see e.g. Jayasena 1999, Burgstaller 2002). Aptamers consist of a random nucleotide sequence flanked on both sides by known sequences that can be used for amplification by PCR. Aptamers have been raised against a variety of molecules such as amino acids, drugs, and proteins. Aptamers with the desired specificity are generally selected in an iterative process, which starts with a library of typically thousands to billions of different aptamers. The library is adsorbed to the target (i.e. CD81 in the context of the present invention) and the non-bound aptamers are washed away and discarded. The bound aptamers are recovered from the target and subsequently amplified by PCR. Then the strands of the amplified (double stranded) aptamers are separated and the enriched pool of aptamers is again adsorbed to the target for a second round of selection. The process is repeated until the desired specificity and affinity is obtained. Finally cloning and sequencing of the enriched aptamers provides unique DNA sequences that may be synthesised and used as CD81 binding agents.

### Small molecules

Libraries of small molecule compounds that can be used as test compounds are available from various commercial suppliers, and they can be made to order using techniques well known in the art, including combinatorial chemistry techniques. Especially in combination with high throughput screening methods, such methods including in particular automated multichannel methods of screening, large libraries of test compounds can be screened according to the methods of the invention. Large libraries can include hundreds, thousands, tens of thousands, hundreds of thousands, and even millions of compounds.

The term "small" molecules as used herein refers to non-polymeric molecules, usually having molecular weights of not more than 2000, more usually molecular weights below 1000, preferably below 500 and most preferably below 300.

Thus in preferred embodiments, the methods for screening test compounds can be performed on a large scale and with high throughput by incorporating, e.g., an array-based assay system and at least one automated or semi-automated step. For example, the assays can be set up using multiple-well plates in which cells are dispensed in individual wells and reagents are added in a systematic manner using a multiwell delivery device suited to the geometry of the multiwell plate. Manual and robotic multiwell delivery devices suitable for use in a high throughput screening assay are well known by those skilled in the art. Each well or array element can be mapped in a one-to-one manner to a particular test condition, such as the test compound. Readouts can also be performed in this multiwell array, preferably using a multiwell plate reader device or the like. Examples of such devices are well known in the art and are available through commercial sources. Sample and reagent handling can be automated to further enhance the throughput capacity of the screening assay, such that dozens, hundreds, thousands, or even millions of parallel assays can be performed in a day or in a week. Fully robotic systems are known in the art for applications such as generation and analysis of combinatorial libraries of synthetic compounds. Alternatively, the binding of a test compound to CD81 can also be determined directly. For example, a radiolabelled test substance can be incubated with CD81 so that binding of the test substance to CD81 can be monitored. For example, the radiolabelled test substance can be incubated with cell membranes containing the polypeptide until equilibrium is reached. The membranes can then be separated from a non-bound test substance and dissolved in scintillation fluid to allow the radioactive content to be determined by scintillation counting. Non-specific binding of the test substance may also be determined by repeating the experiments in the presence of a saturating concentration of a non-radioactive ligand. Preferably, a binding curve is constructed by repeating the experiment with various concentrations of the test substance.

In a preferred embodiment the CD81 binding small molecule is a boraadamantane compound as disclosed in US patent 6613507 B1, which claims the use of such boraadamantane compounds for the treatment of Hepatitis C virus infection.

### Screening of CD81 binding agents for biological effects

Agents that have met the first condition for selection as a CD81 binding agent (i.e. binding to CD81), can subsequently be further screened for their desired effects on the biological activity of CD81 expressing cells. Numerous in vitro screening assays are known in the scientific literature that can be used to test the CD81 binding agents, including assays to determine cellular proliferation, adhesion, motility, secretion of inflammatory mediators and the like. Preferably, the CD81 binding agents are also tested in an in vitro transmigration assay to determine whether and how efficiently they can inhibit or reduce leukocyte transmigration. Said transmigration assay may comprise a continuous layer of endothelial cells and leukocytes or a subpopulation thereof (for example monocytes or T cells) and allows for the quantitative determination of transmigration of leukocytes from one side of the endothelial cell layer to the other side. An example of such a transmigration assay is described in the scientific literature (van der Goes 2001; Floris, 2002).

### Pharmaceutical formulation

Therapeutic formulations of the CD81 binding agents can be prepared by mixing the CD81 binding agent with optional pharmacologically acceptable carriers, excipients or stabilizers, in the form of lyophilised formulations or aqueous solutions. Details on techniques for formulation and administration can be found in the latest edition of Remington's Pharmaceutical Sciences (Mack Publishing, Easton Pa.). Acceptable carriers, excipients, or stabilizers are nontoxic to recipients at the dosages and concentrations employed, and include buffers (such as phosphate); antioxidants; preservatives; carrier proteins (such as serum albumin, gelatin, or immunoglobulins); chelating agents such as EDTA; sugars such as sucrose, mannitol, trehalose or sorbitol; saltforming counter-ions such as sodium; metal complexes (e.g. Zn-protein complexes); and/or non-ionic surfactants such as polyethylene glycol (PEG).

### Treatment with a CD81 binding agent

The pharmaceutical composition containing a CD81 binding agent will be formulated, dosed, and administered in a fashion consistent with good medical practice. Factors for consideration in this context include the particular disease or disorder being treated, the particular mammal being treated, the clinical condition of the individual patient, the cause of the disease or disorder, the method of administration, the scheduling of administration, and other factors known to medical practitioners. The therapeutically effective amount of the CD81 binding agent to be administered will be governed by such considerations.

A therapeutically effective dose refers to that amount of active ingredient which ameliorates the symptoms or condition. Therapeutic efficacy and toxicity can be determined by standard pharmaceutical procedures in cell cultures or with experimental animals, such as by calculating and contrasting the ED50 (the dose therapeutically effective in 50% of the population) and LD50 (the dose lethal to 50% of the population) statistics.

The CD81 binding agent can be administered to a subject by any number of routes including, but not limited to, oral, intravenous, intramuscular, intra-arterial, intramedullary, intrathecal, intraventricular, transdermal, subcutaneous, intraperitoneal, intranasal, enteral, parenteral, topical, sublingual, or rectal means. The preferred route of administration depends in part on the nature of the CD81 binding agent.

A typical daily dose is from about 0.1 to 50 mg per kg of body weight, according to the activity of the compound, the age, weight and conditions of the subject to be treated, the type and severity of the disease and the frequency and route of administration. Preferably, daily dosage levels are from 5 mg to 2 g.

The present invention is illustrated by the following non-limiting example studies.

### Materials and methods used in example studies

### Antibodies

All experiments were performed using the monoclonal antibody AMP-1 which binds to rat CD81. The AMP1 antibody is a mouse IgGlraised by immunizing mice with astrocyte membrane proteins as described in WO 93/10798. The mouse anti-rat VLA-4 antibody TA2, was obtained from Serotec (Oxford, UK). An irrelevant isotype matched control antibody (IgG1) was also obtained from Serotec (Oxford, UK) and used in the experiments as a negative control.

### GP8 endothelial cells

The GP8 endothelial cell line was established from cerebral endothelial cell cultures obtained from Lewis rat brain, by immortalization with the SV40 large T antigen as described previously (Greenwood 1996). GP8 cells retain in culture many of the phenotypic characteristics of in vivo brain endothelial cells, including the expression of Zonula Occludens-1, von Willebrand factor, P-glycoprotein, Glutamate transporter-1 and Intercellular Adhesion Molecule-1 (ICAM-1;). GP8 cells were cultured in Ham's F12 medium (Gibco; Life technologies) supplemented with 20% heat-inactivated FCS (Gibco; Life technologies), 2mM L-Glutamine, 100 U/ml penicillin, and 100 µg/ml streptomycin. For transmigration experiments, GP8 cells were seeded in type I collagen-coated 96-well plates and grown to confluence at 37°C in a 5% CO₂ incubator. The culture medium was replaced every other day until confluence was reached.

### NR8383 monocytes

The rat monocytic cell line NR8383 was obtained from the American Type Culture Collection (#CRL-2192; Manassas, USA) and cultured in RPMI-1640 (BioWhittaker, Oxford, UK) supplemented with 10% heat-inactivated FCS, 2 mM L-glutamine, 100 U/ml penicillin, and 100 µg/ml streptomycin. NR8383 cells were maintained as non-adherent cells in 75 cm² culture flasks in a 5% CO₂ incubator at 37°C.

### Transendothelial migration assay

Leukocyte transmigration was assayed using a well-established a time-lapse videomicroscopy assay that has been described (van der Goes 2001; Floris 2002). Briefly, 50 µl of a 0.5 × 10⁶ cells/ml suspension of NR8383 monocytes was added to 96-well plates containing confluent monolayers of GP8 cerebral endothelial cells. The monocytes were then allowed to settle and migrate over a 4-hour period. At that point, transmigration was monitored by placing the co-cultures in an inverted phase-contrast microscope (Nikon Eclipse TE300) housed in a temperature-controlled (37°C), 5% CO₂ gassed chamber. A microscopic field (200x220 µm) was randomly selected and recorded for 10 minutes at 1/50 of normal speed using a color video 3CCD camera (Sony, with a CMAD2 adapter) coupled to a time-lapse video recorder (Sony SVT S3050P). Tapes were analyzed by enumerating the number of cells within the field that had transmigrated through the monolayer. Transmigrated monocytes could be readily identified because of their phase-dark appearance, whereas adhered monocytes still on top of the endothelial cells had a highly refractive (phase-bright) morphology under phase contrast illumination. The level of transmigration was calculated by dividing the number of transmigrated cells by the total number of monocytes within the field and expressed as a percentage. Data are expressed as the mean ± standard deviation (SD) of 4 individual wells. The statistical significance of differences between group means was determined by Student's t-test.

### Results of example studies

As a first evaluation of the effect of targeting CD81 on leukocyte transmigration , the effect of a anti-rat CD81 antibody (AMP-1) was tested in an in vitro model of leukocyte transmigration as described above. Two different concentrations of antibody were tested (10 µg/ml and 50 µg/ml). As shown in Fig. 1, the highest concentration of AMP-1 (50 µg/ml) resulted in an inhibition of transmigration by 37% (p < 0.01) compared to control IgG1, whereas a lower concentration of AMP-1 (10 µg/ml) resulted in an inhibition of only 21% (p < 0.006). An isotype matched control mAb had no effect at all. It was concluded from this experiments that treatment of endothelial cells and monocytes with different concentrations of AMP-1 reduced monocyte transmigration in a dose-dependent manner, with maximal inhibition at 50 µg/ml.

Next the following question was addressed: since it is known that CD81 is present on the surface of both endothelial cells and monocytes, which of the two cell types is responsible for the inhibitory effect of anti-CD81 antibody on monocyte transmigration observed in Fig. 1? To address this question, the monocytes and endothelial cells were separately preincubated with the anti-CD81 antibody. Before the monocytes were added to the endothelial cells to start transmigration, the antibody was washed away The preincubation was performed with either endothelial cells (EC) only, with monocytes (Mo) only, or with both monocytes and endothelial cells. As shown in Fig. 2, treatment of both EC and MO with 50 µg/ml AMP-1 resulted in an inhibition of transmigration by 55% (p < 0.001) compared to IgG1 treated cells. Preincubation of NR8383 monocytes alone resulted in a similar inhibition (54%: p < 0.001), whereas preincubation of GP8 cells alone inhibited monocyte transmigration to a lesser, but still statistically significant level (37% inhibition; p < 0.005). These results show that (1) a preincubation with antibody is sufficient to reproduce the inhibitory effect observed in Fig. 1 where the antibody is present throughout the transmigration period and (2) the inhibitory effect is predominantly but not exclusively mediated through CD81 on monocytes.

During inflammation in vivo, leukocyte transmigration is usually associated with the expression of activation markers by the endothelium, such as up-regulation of the cell adhesion molecule VCAM-1. As endothelial cell activation directly impacts on leukocyte transmigration we next investigated the effect of pre-activation of the endothelial cells on anti-CD81 mediated inhibition of transmigration (Fig. 3). In this experiment, the endothelial cells were activated with a combination of pro-inflammatory cytokines (interleukin- 1β and interferon-y) for 48 hours prior to the transmigration assay. The assay was performed in the absence or presence of 50 µg/ml AMP-1 (anti-CD81). Furthermore, an antibody against the leukocyte receptor for VCAM-1 (VLA-4) was also included, because it has been described that transmigration inhibition by this antibody is particularly sensitive to the activation state of the endothelium (Floris 2002). Thus, separate wells were assayed in the presence or absence of 10 µg/ml of the anti-VLA-4 antibody TA2. The results are shown in Fig. 3. In the presence of AMP-1, monocyte transmigration across non-stimulated EC was again inhibited by 42 % (p ≤ 0.001). When the endothelial cells were activated with IL-1β and IFN_{Y}, the level of inhibition was even higher (58%; p ≤ 0.001). In the presence of anti-VLA-4, transmigration across activated endothelium was inhibited by 62% (p < 0.001), whereas transmigration across non-stimulated endothelium was not affected. These results demonstrate that anti-CD81-mediated inhibition of monocyte transmigration is even more pronounced on activated EC, and furthermore this inhibition is as strong as anti-VLA4-mediated inhibition.

Taken together, the example studies described above disclose and firmly establish that CD81 is a potent target to inhibit the transmigration of CD81-expressing leukocytes in vitro.

### References

Alon R, Grabovsky V, Feigelson S. Chemokine induction of integrin adhesiveness on rolling and arrested leukocytes local signaling events or global stepwise activation? *Microcirculation.* 2003;10:297-311.
Berditchevski F. Complexes of tetraspanins with integrins: more than meets the eye. *J Cell Sci.* 2001;114:4143-51.
Boucheix C, Rubinstein E. Tetraspanins. *Cell Mol Life Sci.* 2001;58:1189-205.
Cao J, Zhao P, Miao XH, Zhao LJ, Xue LJ, Qi Zt Z. Phage display selection on whole cells yields a small peptide specific for HCV receptor human CD81. *Cell Res.* 2003;13:473-9.
Clark KL, Zeng Z, Langford AL, Bowen SM, Todd SC. PGRL is a major CD81-associated protein on lymphocytes and distinguishes a new family of cell surface proteins. *J Immunol.* 2001;167:5115-21.
Crotta S, Stilla A, Wack A, D'Andrea A, Nuti S, D'Oro U, Mosca M, Filliponi F, Brunetto RM, Bonino F, Abrignani S, Valiante NM. Inhibition of natural killer cells through engagement of CD81 by the major hepatitis C virus envelope protein. *J Exp Med.* 2002 Jan 7;195(1):35-41.
Domanico SZ, Pelletier AJ, Havran WL, Quaranta V. Integrin alpha 6A beta 1 induces CD81-dependent cell motility without engaging the extracellular matrix migration substrate. *Mol Biol Cell.* 1997;8:2253-65.
Feigelson SW, Grabovsky V, Shamri R, Levy S, Alon R. The CD81 tetraspanin facilitates instantaneous leukocyte VLA-4 adhesion strengthening to VCAM-1 under shear flow T. *J Biol Chem.* 2003;278:51203-12.
Floris S, Ruuls SR, Wierinckx A, van der Pol SM, Dopp E, van der Meide PH, Dijkstra CD, De Vries HE. Interferon-beta directly influences monocyte infiltration into the central nervous system. *J Neuroimmunol.* 2002;127:69-79.
Geisert EE Jr, Yang L, Irwin MH. Astrocyte growth, reactivity, and the target of the antiproliferative antibody, TAPA. *J Neurosci.* 1996;16:5478-87. Geisert EE Jr, Williams RW, Geisert GR, Fan L, Asbury AM, Maecker HT, Deng J, Levy S. Increased brain size and glial cell number in CD81-null mice. *J Comp Neurol.* 2002;453:22-32.
Ghosh S, Goldin E, Gordon FH, Malchow HA, Rask-Madsen J, Rutgeerts P, Vyhnalek P, Zadorova Z, Palmer T, Donoghue S; Natalizumab Pan-European Study Group. Natalizumab for active Crohn's disease. *N Engl J Med.* 2003;348:24-32.
Greenwood J, Pryce G, Devine L, Male DK, dos Santos WL, Calder VL, Adamson P. SV40 large T immortalised cell lines of the rat blood-brain and blood-retinal barriers retain their phenotypic and immunological characteristics. *J Neuroimmunol.* 1996;71:51-63.
Hemler ME. Tetraspanin proteins mediate cellular penetration, invasion, and fusion events and define a novel type of membrane microdomain. *Annu Rev Cell Dev Biol.* 2003;19:397-422.
Irwin MH, Geisert EE Jr. The upregulation of a glial cell surface antigen at the astrocytic scar in the rat. *Neurosci Lett.* 1993;154:57-60.
Kelic S, Levy S, Suarez C, Weinstein DE. CD81 regulates neuron-induced astrocyte cell-cycle exit. *Mol Cell Neurosci.* 2001;17:551-60.
Levy S, Todd SC, Maecker HT. CD81 (TAPA-1): a molecule involved in signal transduction and cell adhesion in the immune system. *Annu Rev Immunol.* 1998;16:89-109.
Luscinskas FW, Ma S, Nusrat A, Parkos CA, Shaw SK. The role of endothelial cell lateral junctions during leukocyte trafficking. *Immunol Rev.* 2002;186:57-67.
Mazzocca A, Carloni V, Sciammetta S, Cordella C, Pantaleo P, Caldini A, Gentilini P, Pinzani M. Expression of transmembrane 4 superfamily (TM4SF) proteins and their role in hepatic stellate cell motility and wound healing migration. *J Hepatol.* 2002;37:322-30.
Meerschaert J, Furie MB. The adhesion molecules used by monocytes for migration across endothelium include CD11a/CD18, CD11b/CD18, and VLA-4 on monocytes and ICAM-1, VCAM-1, and other ligands on endothelium. *J Immunol.* 1995;154:4099-112.
Miller DH, Khan OA, Sheremata WA, Blumhardt LD, Rice GP, Libonati MA, Willmer-Hulme AJ, Dalton CM, Miszkiel KA, O'Connor PW; International Natalizumab Multiple Sclerosis Trial Group. A controlled trial of natalizumab for relapsing multiple sclerosis. *N Engl J Med.* 348 (2003) 15-23.
Penas PF, Garcia-Diez A, Sanchez-Madrid F, Yanez-Mo M. Tetraspanins are localized at motility-related structures and involved in normal human keratinocyte wound healing migration. *J Invest Dermatol.* 2000;114:1126-35.
Pileri P, Uematsu Y, Campagnoli S, Galli G, Falugi F, Petracca R, Weiner AJ, Houghton M, Rosa D, Grandi G, Abrignani S. Binding of hepatitis C virus to CD81. *Science.* 1998;282:938-41.
Springer TA. Traffic signals for lymphocyte recirculation and leukocyte emigration: the multistep paradigm. *Cell.* 1994;76:301-14.
Ulbrich H, Eriksson EE and Lindbom L. Leukocyte and endothelial cell adhesion molecules as targets for therapeutic interventions in inflammatory disease. *Trends Pharmacol.Sci.* 2003;24:640-647
VanCompernolle SE, Levy S, Todd SC. Anti-CD81 activates LFA-1 on T cells and promotes T cell-B cell collaboration. *Eur J Immunol.* 2001;31:823-31.
Van der Goes A, Wouters D, Van Der Pol SM, Huizinga R, Ronken E, Adamson P, Greenwood J, Dijkstra CD, De Vries HE. Reactive oxygen species enhance the migration of monocytes across the blood-brain barrier in vitro. *FASEB J.* 2001 Aug;15(10):1852-4.
Wagner CE, Mohler ML, Kang GS, Miller DD, Geisert EE, Chang YA, Fleischer EB, Shea KJ. Synthesis of 1-boraadamantaneamine derivatives with selective astrocyte vs C6 glioma antiproliferative activity. A novel class of anti-hepatitis C agents with potential to bind CD81. *J Med Chem.* 2003;46:2823-33.
Weber C. Novel mechanistic concepts for the control of leukocyte transmigration: specialization of integrins, chemokines, and junctional molecules. *J Mol Med.* 2003;81:4-19.
Yanez-Mo M, Alfranca A, Cabanas C, Marazuela M, Tejedor R, Ursa MA, Ashman LK, de Landazuri MO, Sanchez-Madrid F. Regulation of endothelial cell motility by complexes of tetraspan molecules CD81/TAPA-1 and CD151/PETA-3 with alpha3 betal integrin localized at endothelial lateral junctions. *J Cell Biol.* 1998;141:791-804.
Yednock TA, Cannon C, Fritz LC, Sanchez-Madrid F, Steinman L, Karin N. Prevention of experimental autoimmune encephalomyelitis by antibodies against alpha 4 beta 1 integrin. *Nature.* 1992;356:63-6.
Zhang XA, Bontrager AL, Hemler ME. Transmembrane-4 superfamily proteins associate with activated protein kinase C (PKC) and link PKC to specific beta(1) integrins. *J Biol Chem.* 2001;276:25005-13.

## Claims

1. Use of a CD81 binding agent for manufacturing a medicament for inhibiting or reducing transmigration of cells, such as leukocytes or tumor cells.

2. Use according to claim 1, wherein said CD81 binding agent is selected from the group consisting of CD81-binding polyclonal, monoclonal, chimeric, humanized, and fully human antibodies; CD81-binding Fab, F(ab')₂, single chain Fv or other antibody fragments, CD81-binding CDRs or other CD81-binding peptides, aptamers and small molecules.

3. Use according to claim 1 or 2, wherein said leukocytes are T lymphocytes or monocytes.

4. Use according to any one of claims 1-3, wherein said medicament is for use in the treatment or prevention of (tissue damage caused by) an inflammatory disorder associated with leukocyte transmigration.

5. Use according to claim 4, wherein said disorder is selected from the group consisting of multiple sclerosis, stroke, spinal cord injury, traumatic brain injury, meningitis, Alzheimer's disease, Parkinson's disease, AIDS dementia, atherosclerosis, diabetes, inflammatory bowel disease (including Crohn's disease and ulcerative colitis), ischemia-reperfusion injury (including ischemic stroke and myocard infarction), rheumatoid arthritis, osteoarthritis, psoriasis, complications of tissue or organ transplantation including graft-versus-host and host-versus-graft disease.

6. Use according to any one of claims 1-3, wherein said medicament is for use in the treatment or prevention of tumor metastasis associated with transmigration of tumor cells.

7. A method for inhibiting or reducing transmigration of cells such as leukocytes or tumor cells comprising contacting said cells with a CD81 binding agent.

8. A method according to claim 7, wherein said leukocytes are T lymphocytes or monocytes.

9. A method according to any one of claims 7-8, wherein an effective amount of said CD81 binding agent is administered to a mammal suffering from, or being at risk of developing, (tissue damage caused by) an inflammatory disorder associated with leukocyte transmigration.

10. A method according to claim 9, wherein said disorder is selected from the group consisting of multiple sclerosis, stroke, spinal cord injury, traumatic brain injury, meningitis, Alzheimer's disease, Parkinson's disease, AIDS dementia, atherosclerosis, diabetes, inflammatory bowel disease (including Crohn's disease and ulcerative colitis), ischemia-reperfusion injury (including ischemic stroke and myocard infarction), rheumatoid arthritis, osteoarthritis, psoriasis, complications of tissue or organ transplantation including graft-versus-host and host-versus-graft disease.

11. A method according to any one of claims 7-8, wherein an effective amount of said CD81 binding agent is administered to a mammal suffering from, or being at risk of developing, tumor metastasis associated with transmigration of tumor cells.
